# EUROPÄISCHE PATENTSCHRIFT

(11) **EP 2 687 249 B1**
(45) Veröffentlichungstag und Bekanntmachung des Hinweises auf die Patenterteilung: **14.10.2015**
(21) Anmeldenummer: 13165319.8
(22) Anmeldetag: 25.04.2013
(51) Int. Cl.: A61M 1/16, A61G 12/00, A61G 13/10, H02G 11/00

(54) **Versorgungssystem für Dialysegeräte**
Supply system for dialysis machines
Système d'alimentation pour appareils de dialyse

(30) Priorität: 18.07.2012 DE 102012106494
(43) Veröffentlichungstag der Anmeldung: 22.01.2014
(73) Patentinhaber: DWA Dialyse-Wasser-Aufbereitungsanlagen GmbH & Co. KG, 76698 Ubstadt-Weiher (DE)
(72) Erfinder: Walther, Paul, 76669 Bad Schönborn (DE)
(74) Vertreter: Durm & Partner

(56) Entgegenhaltungen:
- WO-A2-2004/082553
- DE-A1-102008 013 109
- FR-A1- 2 704 150
- US-A- 3 548 122
- US-A1- 2003 080 059

## Beschreibung

Die vorliegende Erfindung betrifft ein Versorgungssystem für Dialysegeräte, umfassend eine Reinwasser führende Ringleitung mit wenigstens einer Entnahmestelle für den Anschluss eines Dialysegeräts, wenigstens eine flexible Abzweigleitung, die von der Entnahmestelle zu dem Dialysegerät führt, und wenigstens ein bewegliches Anschlussterminal, an dem das freie Ende der Abzweigleitung fixiert ist.

Derartige Versorgungssysteme werden in Kliniken bei der Behandlung von Patienten mit Niereninsuffizienz eingesetzt. Die Behandlung erfolgt durch Hämodialyse, wozu große Mengen an Reinwasser, auch Reinstwasser oder Permeat genannt, benötigt werden. Das Reinwasser wird mittels einer Umkehrosmoseanlage erzeugt und über eine Ringleitung zu Entnahmestellen geleitet, an denen die Dialysegeräte angeschlossen werden können. Nicht entnommenes Reinwasser wird zurückgeführt.

Ein solches Versorgungssystem ist beispielsweise aus der DE 10 2008 013 109 A1 bekannt. Es weist eine Reinwasser-Ringleitung auf, die an eine zentrale Wasseraufbereitung angeschlossen ist. Das Reinwasser wird über Abzweigleitungen der Ringleitung entnommen und zu den einzelnen Dialyseplätzen geleitet. An jedem Dialyseplatz befindet sich ein Wandauslass, an dem die Reinwasser-Zu- und -Ableitung angeschlossen werden kann. Es ist ein beweglicher Arm vorgesehen, der die Reinwasser-Zu- und -Ableitung von dem Wandauslass zur Dialysemaschine führt. Der Arm ist fest an der Wand befestigt und weist zwei Gelenke auf, mittels derer eine gewisse Beweglichkeit der Abzweigleitung, vor allem in horizontaler Richtung, ermöglicht wird.

Bei der Behandlung des Patienten kann der für die Hämodialyse benötigte Gefäßzugang (Shunt) sowohl auf der rechten als auch auf der linken Körperseite angeordnet sein. Das Dialysegerät steht zweckmäßigerweise auf derselben Seite wie der Gefäßzugang. Beispielsweise kann bei einem ersten Patienten der Gefäßzugang auf der rechten Seite und bei einem anschließend zu behandelnden zweiten Patienten der Gefäßzugang auf der linken Seite angeordnet sein. Werden nun beide Patienten nacheinander an demselben Dialyseplatz behandelt, ist ein Transport des Dialysegeräts von der rechten auf die linke Patientenseite zwischen den beiden Behandlungen notwendig. Die Position des Wandauslasses ist naturgemäß ortsfest, so dass der Bewegungsbereich des Dialysegeräts von der Länge der durch den Behandlungsraum führenden Abzweigleitungen bestimmt wird. Sind die zu den Behandlungsplätzen führenden Leitungen kurz, ist der Bewegungsbereich klein. Sind die Leitungen aber lang, stellen diese ein Hindernis beim Verschieben oder Verfahren des Dialysegerätes dar und bilden gleichzeitig eine Gefahrenquelle. Außerdem sind frei geführte, lange Abzweigleitungen ein hygienisches Problem.

Angesichts der beschriebenen Problematik ist es Aufgabe der vorliegenden Erfindung, ein Versorgungssystem für Dialysegeräte zu schaffen, das ein einfaches und bequemes Umstellen eines Dialysegerätes z.B. von der einen auf die andere Patientenseite ermöglicht, und bei dem die frei im Raum geführten Leitungen zu den Dialysegeräten möglichst kurz sind.

Bei der Lösung dieser Aufgabe wird ausgegangen von einem Versorgungssystem gemäß dem Oberbegriff des Anspruchs 1. Gelöst wird die Aufgabe durch die im Kennzeichen des Anspruchs 1 angegebenen konstruktiven Merkmale.

Bei dem erfindungsgemäßen Versorgungssystem ist im Raum eine Führungsschiene horizontal angeordnet, entlang der das Anschlussterminal zwischen zwei Endpositionen verfahrbar ist. Die erste Endposition befindet sich in der Nähe der Entnahmestelle und die zweite Endposition weiter davon entfernt; letztere ist durch die Länge der ausgestreckten Abzweigleitung bestimmt. Die Führungsschiene ist als Tragschiene (16) ausgebildet und an einer Wand derart befestigbar, dass sie sich horizontal entlang der Wand erstreckt. Es ist eine Führungseinrichtung vorgesehen, die die Abzweigleitung zumindest abschnittsweise im Wesentlichen parallel zu der Führungsschiene führt und beim Verfahren des Anschlussterminals von der zweiten Endposition in Richtung der ersten Endposition die Abzweigleitung in eine Schlaufe legt.

Die Führungseinrichtung ist in einem Raum zwischen der Wand und dem im Abstand davor angeordneten Anschlussterminal angeordnet. Abhängig von der Länge der Führungsschiene und der Abzweigleitung können somit große Verfahrwege des Anschlussterminals parallel zu der Führungsschiene realisiert werden, ohne dass längere Leitungen frei in den Raum hängen.

Die Führungseinrichtung, welche die Abzweigleitung zumindest abschnittsweise im Wesentlichen parallel zu der Führungsschiene führt, stellt dabei sicher, dass die Abzweigleitung das Verfahren des Anschlussterminals weder behindert noch die Abzweigleitung abgeknickt, gequetscht oder gar beschädigt wird. Indem die Abzweigleitung größtenteils parallel zur Führungsschiene geführt wird, ist es möglich, die Abzweigleitung zum größten Teil unterhalb der Führungsschiene, z.B. an einer Wand, platzsparend anzuordnen.

Erfindungsgemäß kann das Anschlussterminal zwischen einer ersten und einer zweiten Endposition verschoben werden. Die Endpositionen sind dabei so gewählt, dass ein Umstellen eines Dialysegeräts von der einen auf die andere Patientenseite möglich ist, ohne dass die Anschlussleitungen abgekoppelt werden müssen. Beispielsweise wird das mit der Abzweigleitung verbunden Dialysegerät zwei oder drei Meter seitlich verschoben. Die mit dem Gerät ver bundene Abzweigleitung und das Anschlussterminal folgen dieser Verschiebung.

Wird das Anschlussterminal von der am weitesten von der Entnahmestelle entfernten Endposition in Richtung der Entnahmestelle verfahren, wird die Abzweigleitung durch die Führungseinrichtung in eine Schlaufe gelegt. Im Rahmen der vorliegenden Erfindung wird unter einer Schlaufe eine Umlenkung der Abzweigleitung um ungefähr 180° verstanden, so dass die Abzweigleitung eine Bucht bildet. Dabei verläuft die Abzweigleitung in einem ersten Abschnitt ungefähr horizontal in die eine Richtung, bildet in einem zweiten Abschnitt die Bucht mit Umlenkung um ca. 180° und verläuft dann in einem dritten Abschnitt parallel, aber in entgegengesetzter Richtung wie der erste Abschnitt. Dabei verläuft der dritte Abschnitt parallel und mit Abstand zum ersten Abschnitt. Die Abzweigleitung liegt dabei in einer senkrechten Ebene, die sich parallel zur Führungsschiene erstreckt. Die Führung der Abzweigleitung in einer Schlaufe ermöglicht eine platzsparende Unterbringung der Abzweigleitung und bewirkt, dass der in den Raum hineinführende letzte Abschnitt der Leitung nur noch minimale Länge hat, obwohl das Anschlussterminal über eine weite Strecke verfahrbar ist.

In der Tragschiene fährt ein Laufschlitten, welcher das Anschlussterminal trägt. Indem die Tragschiene an einer Wand befestigbar ist, kann das Versorgungssystem einfach in bestehende Räume integriert und platzsparend an der Wand angeordnet werden, gegebenenfalls auch nachträglich. Der Einsatz eines Laufschlittens gewährleistet eine leichte Verfahrbarkeit des Anschlussterminals entlang der Wand.

In bevorzugter Ausführung umfasst die Führungseinrichtung zumindest eine Führungsebene, die unterhalb der Führungsschiene und im Wesentlichen parallel zu dieser angeordnet ist, und auf der in Abhängigkeit der Position des Anschlussterminals ein kürzeres oder längeres Stück der Abzweigleitung auflagert. Diese Führungsebene kann beispielsweise als schmales Brett ausgebildet sein. Indem die Führungsebene unterhalb der Führungsschiene und parallel zu dieser verläuft, ist eine sichere Auflagerung der Abzweigleitung auf der Führungsebene gewährleistet, wodurch Beschädigungen an der Abzweigleitung vermieden werden. Wird das Anschlussterminal von der Entnahmestelle weg in Richtung der äußeren Endposition verfahren, so wird die Schlaufe der Abzweigleitung zurückgebildet, wobei sich ein immer größer werdendes Stück der Abzweigleitung auf der Führungsebene ablegt.

Die Führungseinrichtung kann optional eine zweite Führungsebene oder mehrere Führungsebenen umfassen, die ebenfalls parallel und unterhalb der Führungsschiene verläuft, jedoch mit Abstand über der ersten Führungsebene angeordnet ist, und auf der in Abhängigkeit von der Position des Anschlussterminal ein kleineres oder größeres zweites Stück der Abzweigleitung auflagert. Wird das Anschlussterminal von der zweiten äußeren Endposition zurück in Richtung der ersten Endposition verfahren, legt sich die Abzweigleitung in eine Schlaufe. Die Abzweigleitung liegt dann in einem kürzer werdenden ersten Abschnitt auf der ersten Führungsebene auf, verläuft in einem zweiten Abschnitt in der Schlaufe und wird im anschließenden dritten Abschnitt von der zweiten Führungsebene aufgenommen. Mithilfe der Schlaufe wird der Höhenunterschied zwischen der ersten und zweiten Führungsebene überwunden.

Die zweite Führungsebene kann ebenfalls als schmales Brett ausgebildet sein. Es versteht sich aber, dass die Führungsebenen nicht unbedingt durchgehend ausgebildet sein müssen, sondern z.B. aus mehreren, voneinander beabstandeten Auflagestellen für die Abzweigleitung bestehen können.

Umfasst die Führungseinrichtung mindestens einen flexiblen Stützschlauch, welcher die mindestens eine Abzweigleitung umhüllt, wird ein seitliches Ausweichen oder gar Abknicken der Abzweigleitung verhindert. Somit wird ein reibungsloser Bewegungsablauf gewährleistet sowie Beschädigungen an der Abzweigleitung vermieden.

In vorteilhafter Weiterbildung der Erfindung hat der Stützschlauch eine gewisse Steifigkeit, so dass begrenzt Druckkräfte in Verfahrrichtung des Anschlussterminals übertragbar sind. Befindet sich das Anschlussterminal beispielsweise in der ersten Endposition und wird in Richtung der zweiten Endposition verfahren, wirken ausgehend von dem Anschlussterminal Druckkräfte auf den Stützschlauch. Diese bewirken ein Verschieben der Abzweigleitung auf der zweiten Führungsebene. Dadurch verkürzt sich das auf der zweiten Führungsebene liegende Teilstück des Stützschlauchs. Die begrenzte Steifigkeit des Stützschlauchs unterstützt die Bildung einer Schlaufe in der Abzweigleitung zwischen der ersten und zweiten Führungsebene.

Vorteilhaft hat das Anschlussterminal eine im Wesentlichen vertikale Frontplatte und ist das freie Ende der Abzweigleitungen durch eine Durchführung oder eine Öffnung in der Frontplatte hindurch geführt. Beispielsweise führt die Führungseinrichtung die Abzweigleitung bis an die Rückseite der Frontplatte heran. Um die Abzweigleitung weiter von der Rückseite auf die Vorderseite der Frontplatte zu führen, dient die Durchführung in der Frontplatte. Auf diese Weise kann die Abzweigleitung in einem Stück von der Entnahmestelle bis zum Dialysegerät ausgeführt sein, ohne jede Verbindungsstelle oder Zwischenkupplung.

Bevorzugt sind parallel zu der Abzweigleitung weitere Zu- und/oder Ableitungen, insbesondere für Dialysekonzentrat und/oder Dialysat, in dem Stützschlauch geführt. Somit können alle für die Hämodialyse benötigten Flüssigkeiten über dasselbe Anschlussterminal zu- und abgeführt werden. Darüber hinaus entfallen gesonderte Führungseinrichtungen für weitere Zu- und/oder Ableitungen.

Bei der Blutwäsche müssen zur Vermeidung von Infektionen sehr hohe Hygienestandards erfüllt werden. Zweckmäßig ist deshalb die komplette Führungseinrichtung hinter einer Verkleidung angeordnet. Damit ist die Führungseinrichtung gegen Verschmutzung und auch gegen Beschädigung geschützt. Ist die Verkleidung abnehmbar ausgeführt, bleibt die komplette Führungseinrichtung einschließlich Schläuche und Leitungen für Wartungszwecke oder Reparaturarbeiten zugänglich.

Bevorzugt sind mehrere Anschlussterminals entlang derselben Führungsschiene verfahrbar, wobei jedem Anschlussterminal eine Führungseinrichtung zugeordnet ist. Somit können in einem Behandlungsraum mehrere Dialysegeräte angeschlossen und mehrere Patienten gleichzeitig behandelt werden. Die vorhandenen Räumlichkeiten können effizienter genutzt werden. Durch die stufenlos verfahrbaren Anschlussterminals können die Behandlungsplätze enger oder bei Bedarf auch mit größerem Abstand nebeneinander angeordnet werden, ohne dass an der Installation etwas geändert werden muss.

Ein Ausführungsbeispiel der Erfindung wird nachstehend anhand der beigefügten Zeichnungen beschrieben. Es zeigen:
- Figur 1: ein System zur Versorgung von Dialysegeräten mit Reinwasser, in einem Prinzipbild;
- Figur 2: ein Versorgungssystem, das teilweise hinter einer Verkleidung verborgen ist, mit einem Anschlussterminal für ein Dialysegerät, in einer perspektivischen Ansicht;
- Figur 3: einen Vertikalschnitt entlang der Schnittlinie A-A in Figur 2;
- Figur 4: das Versorgungssystem von Figur 2 bei abgenommener Verkleidung;
- Figur 5: das Versorgungssystem wie in Figur 4, mit nach rechts gefahrenem Anschlussterminal.

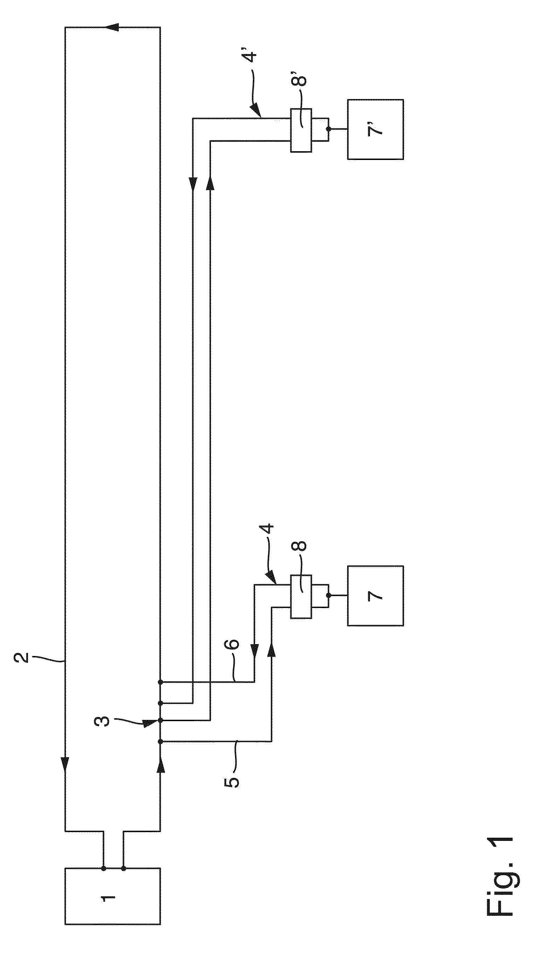

Das Prinzipbild von Fig. 1 beschränkt sich auf die Einrichtungen zur Versorgung eines oder mehrerer Dialysegeräte mit Reinwasser. Es ist aber klar, dass ein Dialysegerät zur Durchführung der Hämodialyse auch mit Dialysekonzentrat versorgt werden muss, und dass das Gerät ferner an das Stromnetz und gegebenenfalls weitere Ver- und Entsorgungsleitungen angeschlossen werden muss.

Das benötigte Reinwasser wird in einer Umkehrosmoseanlage 1 erzeugt und in einer angeschlossenen Ringleitung 2 im Kreis geführt, so dass nicht verbrauchtes Reinwasser zur Umkehrosmoseanlage 1 zurückgeführt wird. In der Ringleitung 2 sind Entnahmestellen 3 vorgesehen, an denen eine oder mehrere Abzweigleitungen 4 angeschlossen sind. Die Ringleitung 2 ist stationär und in der Regel Bestandteil des Gebäudes, in dem sich der Behandlungsraum für die Dialysepatienten befindet. Auch die Umkehrosmoseanlage 1 befindet sich üblicherweise außerhalb des Behandlungsraums. Die Entnahmestelle 3 kann vielfach, insbesondere einfach, doppelt oder gar dreifach ausgebildet sein, so dass an einer bestimmten Stelle der Ringleitung 2 eine entsprechende Anzahl von Abzweigleitungen 4 für die Versorgung mehrerer Behandlungsplätze anschließbar ist.

Die Abzweigleitungen 4 bestehen aus zwei flexiblen Schläuchen, die parallel laufen, und umfassen jeweils eine Zuleitung 5 und eine Rückleitung 6. Das zu versorgende Dialysegerät 7 ist zwischen Zuleitung 5 und Rückleitung 6 angeschlossen, so dass nicht entnommenes Reinwasser durch die Rückleitung 6 zurück in die Ringleitung 2 geführt werden kann. Ein zweites Dialysegerät 7' ist über eine zweite Abzweigleitung 4' mit der Ringleitung 2 verbunden. Wenn, wie in diesem Beispiel, das zweite Dialysegerät 7' weiter von der Entnahmestelle 3 entfernt aufgestellt ist als das erste Dialysegerät 7, so muss die Abzweigleitung 4', die zu dem zweiten Dialysegerät 7' führt, länger sein als die erste Abzweigleitung 4 zu dem ersten Dialysegerät 7.

Jede Abzweigleitung 4, 4' ist an einem beweglichen Anschlussterminal 8, 8' fixiert. Eine Verschiebung oder Umstellung der Dialysegeräte 7, 7' im Behandlungsraum ist nur möglich, wenn auch das zugehörige Anschlussterminal 8 bzw. 8' verfahren wird, z.B. entlang einer Wand des Behandlungsraums von rechts nach links oder umgekehrt. Die maximale Entfernung der Dialysegeräte 7, 7' von der Entnahmestelle 3 ist dabei von der Länge der zugehörigen Abzweigleitung 4 bzw. 4' vorgegeben.

Im Folgenden wird nun die konstruktive Ausgestaltung des Anschlussterminals 8 sowie der zugehörigen Einrichtungen für die Führung der Abzweigleitung 4 von der Entnahmestelle 3 bis zum Dialysegerät 7 beschrieben.

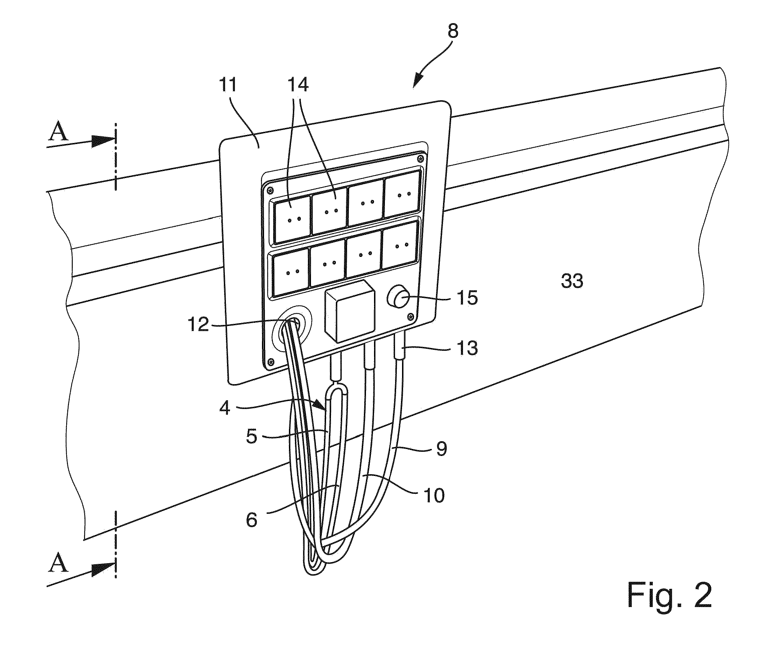

Wie in Fig. 2 zu sehen, führt das Anschlussterminal 8 nicht nur die Abzweigleitung 4 für Reinwasser, sondern zusätzlich auch eine Dialysekonzentratleitung 9 und eine Dialysatabflussleitung 10, welche gleichfalls an das (hier nicht dargestellte) Dialysegerät angeschlossen werden müssen. Über die Dialysekonzentratleitung 9 wird dem Dialysegerät eine konzentrierte Dialyseflüssigkeit zugeführt, die im Innern des Geräts mit dem Reinwasser, das durch die Abzweigleitung 4 zufließt, verdünnt wird. Verbrauchte Dialyseflüssigkeit, die mit Schadstoffen aus dem gereinigten Blut des Patienten angereichert ist, fließt durch die Dialysatabflussleitung 10 ab und wird verworfen. Alle drei Leitungen 4, 9 und 10 müssen also mitgeführt werden, wenn sich die Aufstellung des Dialysegeräts im Behandlungsraum ändert.

Das Anschlussterminal 8 hat eine vertikale Frontplatte 11, in der eine runde Durchführung 12 vorgesehen ist. Durch diese Durchführung 12 sind insgesamt vier einzelne Leitungen hindurchgeführt: Zuleitung 5 und Rückleitung 6 der Abzweigleitung 4 für Reinwasser, Dialysekonzentratleitung 9 sowie Dialysatabflussleitung 10. Die freien Enden dieser Leitungen hängen nach vorne heraus. Die Abzweigleitung 4 endet in einem T-Stück, das Zuleitung 5 und Rückleitung 6 verbindet. Alleine durch ihr Eigengewicht sind die Leitungen 5, 6, 9, 10 lose an der Frontplatte 11 des Anschlussterminals 8 fixiert. Bei Nichtgebrauch, also abgekoppeltem Dialysegerät, können die Leitungen 5, 6, 9 und 10 auf Halterungen 13 aufgesteckt werden, welche an der Unterseite der Frontplatte 11 angeordnet sind. Dadurch werden die freien Enden und insbesondere die offenen Anschlussstücke der Versorgungsleitungen vom Boden fern gehalten und Kontaminierung wird vorgebeugt.

In der Frontplatte 11 des Anschlussterminals 8 sind überdies Steckdosen 14 eingebaut, die dem elektrischen Anschluss des Dialysegeräts sowie gegebenenfalls von weiteren Analyse-Kontroll- und Behandlungsgeräten dienen. Die zugehörigen elektrischen Leitungen werden von der Rückseite der Frontplatte 11 zugeführt. Optional ist ferner ein Konzentratwahlschalter 15 in die Frontplatte 11 integriert, über den verschiedene Dialysekonzentrate ausgewählt werden können. Das ausgewählte Dialysekonzentrat wird dann über die Dialysekonzentratleitung 9 bereitgestellt.

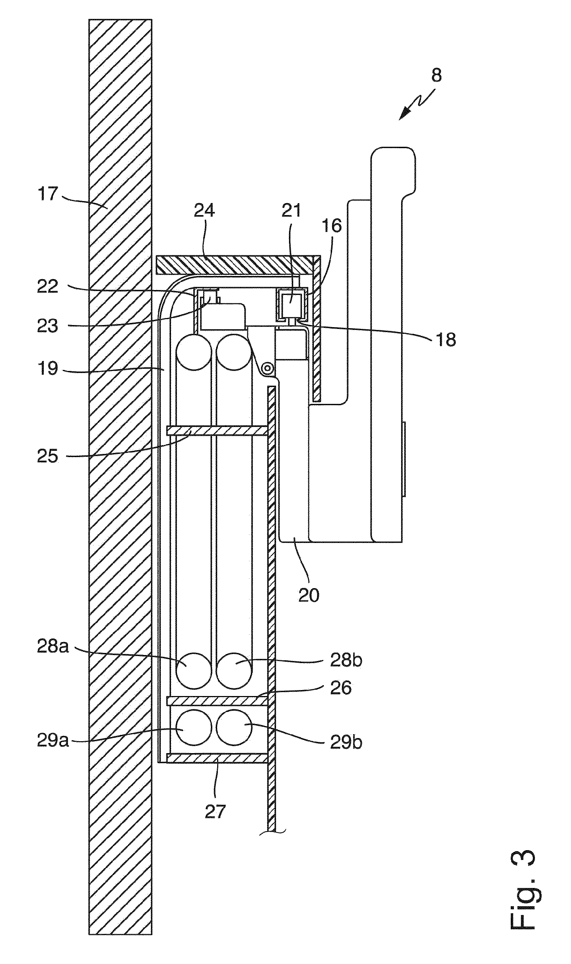

In dem Schnittbild von Fig. 3 ist erkennbar, wie das Anschlussterminal 8 so gelagert ist, dass es in horizontaler Richtung verfahrbar ist, wobei sämtliche hydraulischen und elektrischen Leitungen von und zu dem zugehörigen Dialysegerät mitgeführt werden müssen.

Eine als Führungsschiene dienende Tragschiene 16 erstreckt sich horizontal entlang der Wand 17 des Behandlungsraums. Die Tragschiene 16 ist als Hohlprofil mit ungefähr quadratischem Querschnitt ausgebildet und hat an ihrer Unterseite einen Führungsschlitz 18. Mittels L-förmig ausgebildeter Montagewinkel 19 ist die Tragschiene 16 an der Wand 17 befestigt.

Das Anschlussterminal 8 sitzt auf einem Laufschlitten 20, an dessen Oberseite Gleitlager 21 vorgesehen sind. Diese Gleitlager 21 gleiten auf der Tragschiene 16, so dass der Laufschlitten 20 mitsamt dem daran befestigten Anschlussterminal 8 entlang der Tragschiene 16 hin- und herfahren kann. Eine parallel zur Tragschiene 16 verlaufende Winkelschiene 22 ist ebenfalls mittels der Montagewinkel 19 an der Wand 17 befestigt. Zusätzliche Lagerrollen 23 des Laufschlittens 20 laufen auf dieser Winkelschiene 22 und stützen den Laufschlitten 20 ab.

Eine im Profil L-förmige Abdeckung 24 schützt die Tragschiene 16, die Winkelschiene 22 und den oberen Teil des Laufschlittens 20 gegen Verschmutzung und ungewollten Eingriff. Aus der Sicht des Bedieners bzw. Patienten schwebt das Anschlussterminal 8 scheinbar vor der Wand 17.

In dem Raum zwischen Wand 17 und dem im Abstand davor angeordneten Anschlussterminal 8 ist eine Führungseinrichtung für alle hydraulischen und elektrischen Leitungen angeordnet, welche beim horizontalen Verfahren des Anschlussterminals 8 mitgeführt werden müssen. Diese Führungseinrichtung umfasst eine obere Führungsebene 25 und eine untere Führungsebene 26. Die obere Führungsebene 25 verläuft in relativ geringem Abstand unterhalb und im Wesentlichen parallel zu der Tragschiene 16. Die untere Führungsebene 26 verläuft ebenfalls parallel, jedoch in größerem Abstand zu der Tragschiene 16. Die Führungsebenen 25, 26 können beispielsweise als schmale Bretter ausgebildet sein, die punktuell an den Montagewinkeln 19 befestigt sind.

Die Führungseinrichtung umfasst ferner zwei parallele Stützschläuche 28a und 28b. Im Innern des einen Stützschlauchs 28a laufen die flüssigkeitsführenden Leitungen, also die Abzweigleitung 4 für Reinwasser, die Dialysekonzentratleitung 9 und die Dialysatabflussleitung 10 (vgl. Fig. 2). Der zweite Stützschlauch 28b umgibt die elektrischen Leitungen, die z.B. zu den Steckdosen 14 und dem Konzentratwahlschalter 15 auf der Frontplatte 11 des Anschlussterminals 8 führen (vgl. Fig. 2).

Unterhalb der unteren Führungsebene 26 verläuft noch eine weitere Führungsebene 27, die der Auflagerung eines zweiten Paares von Stützschläuchen 29a, 29b dient. Diese weiteren Stützschläuche 29a, 29b umhüllen die Fluidleitungen bzw. elektrischen Leitungen, die zu einem weiteren (nicht dargestellten) Anschlussterminal gehören.

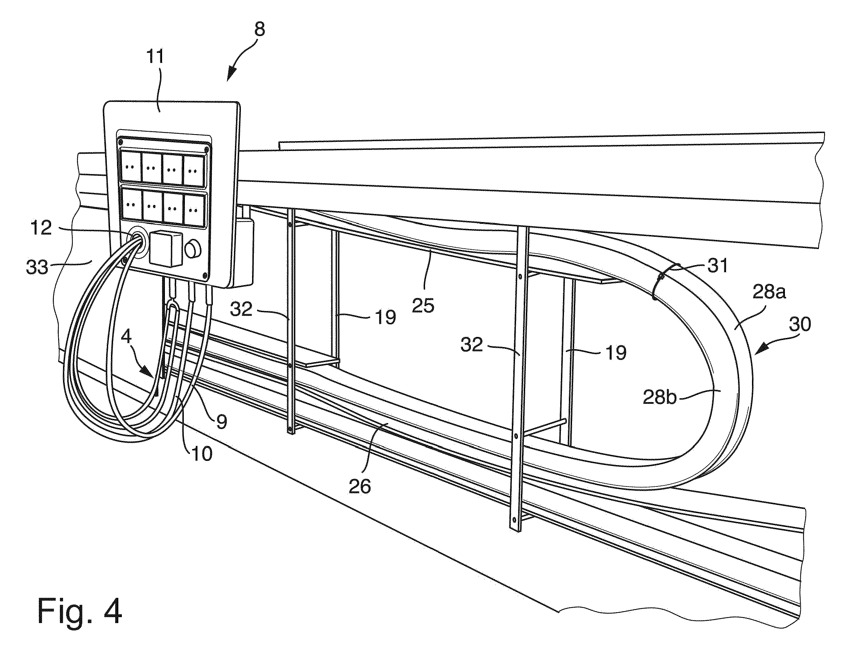

Fig. 4 lässt die Führung der Leitungen innerhalb und außerhalb der Stützschläuche 28a, 28b erkennen. Das Anschlussterminal 8 befindet sich hier in einer ersten Endposition, die der Entnahmestelle 3 der Ringleitung 2 (vgl. Fig. 1) relativ nahe liegt. Die Abzweigleitung 4, die Dialysekonzentratleitung 9 und die Dialysatabflussleitung 10 verschwinden in der Durchführung 12 der Frontplatte 11. Hinter der Frontplatte 11 - und somit in Fig. 4 unsichtbar - laufen die Leitungen 4, 9 und 10 in den Stützschlauch 28a ein. Die elektrischen Leitungen führen von der Rückseite der Frontplatte 11 in den parallelen zweiten Stützschlauch 28b. Da die Stützschläuche 28a und 28b somit die in ihrem Inneren geführten Leitungen umgeben, ist deren weiterer Verlauf in Fig. 4 unsichtbar. Der Verlauf der Stützschläuche 28a und 28b lässt aber den Verlauf der in ihrem Inneren geführten Leitungen 4, 9, 10 bzw. der Elektroleitungen erkennen.

In einem ersten Abschnitt, der beim Anschlussterminal 8 beginnt, verläuft der Stützschlauch 28a, der die Fluidleitungen 4, 9, 10 führt, in etwa parallel und knapp unterhalb der Tragschiene 16. Dieser Abschnitt des Stützschlauches 28a lagert dabei auf der oberen Führungsebene 25 auf. Es schließt sich ein halbkreisförmig gebogener Abschnitt an, in dem der Stützschlauch 28a um ungefähr 180° umgelenkt wird. Der folgende dritte Abschnitt des Stützschlauchs 28a verläuft wiederum parallel zu, aber in erheblich größerem Abstand unterhalb der Tragschiene 16. Der Stützschlauch 28a bildet also eine Schlaufe 30, die sich auf der unteren Führungsebene 26 abstützt. Von der Schlaufe 30 führt der Stützschlauch 28a dann entlang der unteren Führungsebene 26 in Richtung der Entnahmestelle (vgl. Fig. 1).

Der zweite Stützschlauch 28b, der die Elektroleitungen führt, verläuft parallel zum Stützschlauch 28a für die Fluidleitungen.

Die Stützschläuche 28a, 28b bestehen aus flexiblem Kunststoff, weisen aber gleichzeitig eine begrenzte Steifigkeit auf, so dass sie Druckkräfte in Längsrichtung aufnehmen und übertragen können. Erreicht wird das beispielsweise dadurch, dass die Schlauchwand profiliert ist und starre Segmente mit flexiblen Segmenten abwechseln, so ähnlich wie bei einem Staubsaugerschlauch.

Die beiden parallelen Stützschläuche 28a und 28b sind in regelmäßigen Abständen miteinander verbunden durch Kabelbinder 31. Dadurch stützen sich die Schläuche 28a, 28b gegenseitig, wird ein Verdrehen verhindert und insbesondere die Auflagerung auf den Führungsebenen 25 und 26 verbessert. Anstelle von Kabelbindern 31 können selbstverständlich auch andere geeignete Verbindungsmittel wie Drahtschlaufen, Clips usw. verwendet werden.

Die oberen und unteren Führungsebenen 25, 26 sind hier als schmale dünne Bretter aus Holz oder Holzwerkstoff ausgebildet. Es sind aber auch andere Materialien denkbar, wobei darauf geachtet werden muss, dass die Stützschläuche 28a, 28b auf den Oberseiten der Führungsebenen 25, 26 gut gleiten. Streben 32 aus Metall halten die Führungsebenen 25, 26 in ihrer vorgegebenen Position. Diese Streben 32 tragen auch ein Verkleidungspaneel 33, das mehrteilig ausgebildet ist. Das Verkleidungspaneel 33 kann beispielsweise aus Holz- oder Kunststoffplatten bestehen. In Fig. 4 ist der vordere Teil des Verkleidungspaneels 33 abgenommen, in Fig. 2 ist die Verkleidung komplett.

Wird nun das Anschlussterminal 8 von der einen Endposition entsprechend Fig. 4 nach rechts, also weiter weg von der Entnahmestelle, verschoben, so rutschen die Stützschläuche 28a, 28b auf der oberen Führungsebene 25 in dieselbe Richtung, in der Abbildung also nach rechts. Aufgrund der begrenzten Steifigkeit der Stützschläuche 28a, 28b wird auch die Schlaufe 30 nach rechts verlagert. Dadurch lagern sich die Stützschläuche 28a, 28b mit einem immer länger werdenden Stück auf die untere Führungsebene 26 auf.

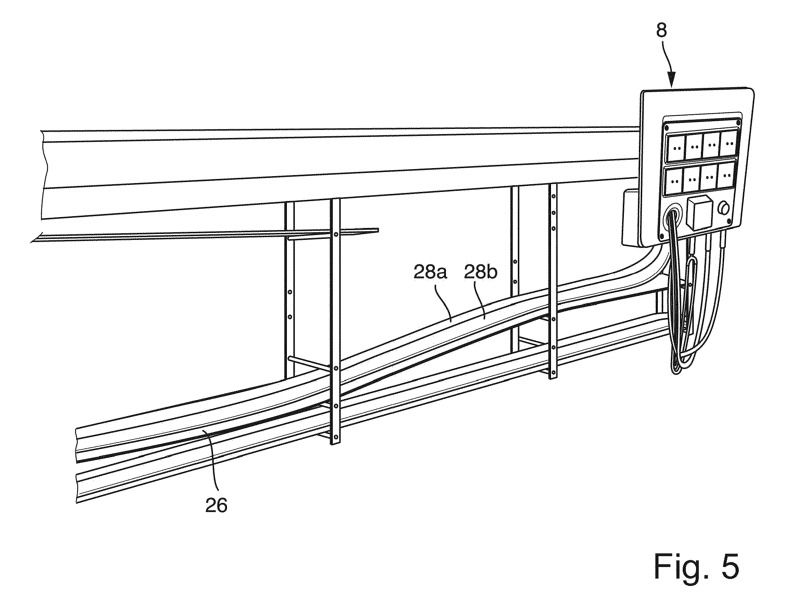

Fig. 5 zeigt die Endposition des Anschlussterminals 8, bei der sich dieses in maximaler Entfernung von der Entnahmestelle 3 befindet. Die Stützschläuche 28a, 28b haben sich entrollt und liegen jetzt ausgestreckt fast vollständig auf der unteren Führungsebene 26 auf. Die Länge der ausgestreckten Stützschläuche 28a, 28b bzw. die Länge der darin geführten Leitungen 4, 5, 6, 9, 10 bestimmt die zweite Endposition des Anschlussterminals 8, also die maximale Entfernung von der Entnahmestelle 3.

Wird das Anschlussterminal 8 aus der in Fig. 5 dargestellten zweiten Endposition wieder zurück in Richtung der ersten Endposition - nach links - verfahren, so legen sich die Stützschläuche 28a, 28b und damit auch die im Inneren geführten Leitungen, insbesondere die Abzweigleitung 4 für Reinwasser, wieder in eine Schlaufe 30, wie in Fig. 4 zu sehen. Dieses Auf- und Abrollen, Auflagern und Abheben bzw. Verschieben der Stützschläuche 28a, 28b und der darin geführten Leitungen geschieht dabei in einem Raum hinter dem Anschlussterminal 8 und ist dadurch dem Betrachter verborgen, wenn das Verkleidungspaneel 33 montiert ist (vgl. Fig. 2).

### Bezugszeichen

- 1: Umkehrosmoseanlage
- 2: Ringleitung
- 3: Entnahmestelle
- 4, 4': Abzweigleitungen
- 5: Zuleitung (von 4)
- 6: Rückleitung (von 5)
- 7, 7': Dialysegeräte
- 8, 8': Anschlussterminals

- 9: Dialysekonzentratleitung
- 10: Dialysatabflussleitung
- 11: Frontplatte
- 12: Durchführung (in 11)
- 13: Halterungen
- 14: Steckdosen
- 15: Konzentratwahlschalter

- 16: Tragschiene
- 17: Wand
- 18: Führungsschlitz (in 16)
- 19: Montagewinkel
- 20: Laufschlitten
- 21: Gleitlager (von 20)
- 22: Winkelschiene
- 23: Lagerrolle (an 20)
- 24: Abdeckung
- 25: obere Führungsebene
- 26: untere Führungsebene
- 27: weitere Führungsebene
- 28a, 28b: Stützschläuche
- 29a, 29b: weitere Stützschläuche
- 30: Schlaufe
- 31: Kabelbinder
- 32: Streben
- 33: Verkleidungspaneel

## Patentansprüche

1. Versorgungssystem für Dialysegeräte, umfassend
eine Reinwasser führende Ringleitung (2) mit wenigstens einer Entnahmestelle (3) für den Anschluss eines Dialysegeräts (7);
wenigstens eine flexible Abzweigleitung (4), die von der Entnahmestelle (3) zu dem Dialysegerät (7) führt;
wenigstens ein bewegliches Anschlussterminal (8), an dem das freie Ende der Abzweigleitung (4) fixiert ist;
**gekennzeichnet durch**
eine horizontal angeordnete, als Tragschiene (16) ausgebildete Führungsschiene, entlang der das Anschlussterminal (8) zwischen zwei Endpositionen verfahrbar ist, wobei sich die erste Endposition in der Nähe der Entnahmestelle (3) befindet und die zweite Endposition **durch** die Länge der ausgestreckten Abzweigleitung (4) bestimmt ist;
wobei die Tragschiene (16) an einer Wand derart befestigbar ist, dass sie sich horizontal entlang der Wand erstreckt,
eine Führungseinrichtung, welche die Abzweigleitung (4) zumindest abschnittsweise im Wesentlichen parallel zu der Tragschiene (16) führt und beim Verfahren des Anschlussterminals (8) von der zweiten Endposition in Richtung der ersten Endposition die Abzweigleitung (4) in eine Schlaufe (30) legt;
wobei die Führungseinrichtung in einem Raum zwischen der Wand und dem im Abstand davor angeordneten Anschlussterminal angeordnet ist.

2. Versorgungssystem nach Anspruch 1, **dadurch gekennzeichnet, dass** auf der Tragschiene (16) ein Laufschlitten (20) fährt, welcher das Anschlussterminal (8) trägt.

3. Versorgungssystem nach Anspruch 2, **dadurch gekennzeichnet, dass** die Führungseinrichtung wenigstens eine Führungsebene (25) umfasst, die unter der Tragschiene (16) und im Wesentlichen parallel zu dieser angeordnet ist und auf der in Abhängigkeit der Position des Anschlussterminals (8) ein kürzeres oder längeres Stück der Abzweigleitung (4) auflagert.

4. Versorgungssystem nach Anspruch 3, **dadurch gekennzeichnet, dass** die Führungseinrichtung eine zweite Führungsebene (26) umfasst, die ebenfalls parallel und unter der Tragschiene (16) verläuft, jedoch mit Abstand unter der ersten Führungsebene (25) angeordnet ist und auf der in Abhängigkeit von der Position des Anschlussterminals (8) ein kleineres oder größeres zweites Stück der Abzweigleitung (4) auflagert.

5. Versorgungssystem nach einem der Ansprüche 1 bis 4, **dadurch gekennzeichnet, dass** die Führungseinrichtung mindestens einen flexiblen Stützschlauch (28a) umfasst, welcher die mindestens eine Abzweigleitung (4) umgibt.

6. Versorgungssystem nach Anspruch 5, **dadurch gekennzeichnet, dass** der Stützschlauch (28a) eine begrenzte Steifigkeit aufweist, so dass er Druckkräfte in Längsrichtung aufnehmen und übertragen kann.

7. Versorgungssystem nach einem der Ansprüche 1 bis 6, **dadurch gekennzeichnet, dass** das Anschlussterminal (8) eine im Wesentlichen vertikale Frontplatte (11) hat und das freie Ende der Abzweigleitung (4) durch eine Durchführung (12) in der Frontplatte (11) hindurch geführt ist.

8. Versorgungssystem nach einem der Ansprüche 5 bis 7, **dadurch gekennzeichnet, dass** parallel zu der Abzweigleitung (4) weitere Zu- und/oder Ableitungen, insbesondere für Dialysat und/oder Dialysekonzentrat, in dem Stützschlauch (28a) geführt sind.

9. Versorgungssystem nach einem der Ansprüche 1 bis 8, **dadurch gekennzeichnet, dass** die Führungseinrichtung hinter einer abnehmbaren Verkleidung (33) angeordnet ist.

10. Versorgungssystem für Dialysegeräte nach einem der Ansprüche 1 bis 9, **dadurch gekennzeichnet, dass** mehrere Anschlussterminals (8, 8') entlang derselben Tragschiene (16) verfahrbar sind,
jedem Anschlussterminal (8, 8') eine Führungseinrichtung zugeordnet ist, welche zumindest die zugehörige Abzweigleitung (4, 4') für Reinwasser führt.

## Claims

1. A supply system for dialysis machines, comprising
a pure water guiding ring line (2) having at least one tapping point (3) for the connection of a dialysis machine (7);
at least one flexible branch line (4), which leads from the tapping point (3) to the dialysis machine (7);
at least one movable connection terminal (8), to which the free end of the branch line (4) is fixed;
**characterised by**
a horizontally arranged guide rail formed as a supporting rail (16), along which guide rail the connection terminal (8) can be moved between two end positions, wherein the first end position is located in the vicinity of the tapping point (3) and the second end position is determined by the length of the outstretched branch line (4);
wherein the supporting rail (16) can be secured to a wall such that it extends horizontally along the wall,
a guide device, which guides the branch line (4) at least in portions substantially parallel to the supporting rail (16) and forms a loop (30) as the connection terminal (8) is moved from the second end position in the direction of the first end position;
wherein the guide device is arranged in a space between the wall and the connection terminal arranged at a distance in front of said wall.

2. The supply system according to Claim 1, **characterised in that**
a slide (20) travels on the supporting rail (16) and carries the connection terminal (8).

3. The supply system according to Claim 2, **characterised in that** the guide device comprises at least one guide level (25), which is arranged below the supporting rail (16) and substantially parallel thereto and supports a piece of the branch line (4), which piece is shorter or longer depending on the position of the connection terminal (8).

4. The supply system according to Claim 3, **characterised in that** the guide device comprises a second guide level (26), which also runs parallel to and below the supporting rail (16), but is arranged at a distance below the first guide level (25), and on which a second piece of the branch line (4) is supported, which piece is shorter or longer depending on the position of the connection terminal (8).

5. The supply system according to one of Claims 1 to 4, **characterised in that** the guide device comprises at least one flexible supporting tube (28a), which surrounds the at least one branch line (4).

6. The supply system according to Claim 5, **characterised in that** the supporting tube (28a) has a limited rigidity, such that it can absorb and transfer compressive forces in the longitudinal direction.

7. The supply system according to one of Claims 1 to 6, **characterised in that** the connection terminal (8) has a substantially vertical front plate (11) and the free end of the branch line (4) is guided through a feedthrough (12) in the front plate (11).

8. The supply system according to one of Claims 5 to 7, **characterised in that** further feed and/or discharge lines, in particular for dialysate and/or dialysis concentrate, is/are guided in the supporting tube (28a) parallel to the branch line (4).

9. The supply system according to one of Claims 1 to 8, **characterised in that** the guide device is arranged behind a removable covering (33).

10. The supply system for dialysis machines according to one of Claims 1 to 9, **characterised in that** a number of connection terminals (8, 8') can be moved along the same supporting rail (16),
each connection terminal (8, 8') is assigned a guide device, which at least guides the associated branch line (4, 4') for clean water.

## Revendications

1. Système d'alimentation pour appareils de dialyse, comprenant
une conduite circulaire (2) amenant de l'eau pure, avec au moins un point de prélèvement (3) pour le raccordement d'un appareil de dialyse (7) ;
au moins une conduite de dérivation flexible (4), allant du point de prélèvement (3) à l'appareil de dialyse (7) ;
au moins un terminal de raccordement mobile (8), au niveau duquel est fixée l'extrémité libre de la conduite de dérivation (4) ;
**caractérisé par**
un rail de guidage agencé horizontalement, réalisé en tant que rail de support (16), le long duquel le terminal de raccordement (8) est roulant entre deux positions d'extrémité, dans lequel la première position d'extrémité se trouve à proximité du point de prélèvement (3) et la seconde position d'extrémité est déterminée par la longueur de la conduite de dérivation (4) étirée ;
dans lequel le rail de support (16) peut être fixé sur une paroi de sorte qu'il s'étend horizontalement le long de la paroi,
un dispositif de guidage, qui guide la conduite de dérivation (4) au moins par section de manière essentiellement parallèle au rail de support (16) et dépose la conduite de dérivation (4) dans une boucle (30)lors du roulage du terminal de raccordement (8) depuis la seconde position d'extrémité vers la première position d'extrémité ;
dans lequel le dispositif de guidage est agencé dans un espace situé entre la paroi et le terminal de raccordement agencée espacée devant celle-ci.

2. Système d'alimentation selon la revendication 1, **caractérisé en ce qu'**un chariot roulant (20), qui porte le terminal de raccordement (8), se déplace sur le rail de support (16).

3. Système d'alimentation selon la revendication 2, **caractérisé en ce que** le dispositif de guidage comprend au moins un plan de guidage (25), qui est agencé sous le rail de support (13) et est essentiellement parallèle à celui-ci et sur lequel repose une partie plus courte ou plus longue de la conduite de dérivation (4) en fonction de la position du terminal de raccordement (8).

4. Système d'alimentation selon la revendication 3, **caractérisé en ce que** le dispositif de guidage comprend un second plan de guidage (26), qui passe également de manière parallèle au, et sous le, rail de support (16), mais est cependant agencé espacé sous le premier plan de guidage (25) et sur lequel repose une seconde partie plus petite ou plus grande de la conduite de dérivation (4) en fonction de la position du terminal de raccordement (8).

5. Système d'alimentation selon l'une quelconque des revendications 1 à 4, **caractérisé en ce que** le dispositif de guidage comprend au moins un tuyau d'appui flexible (28a), qui entoure la au moins une conduite de dérivation (4).

6. Système d'alimentation selon la revendication 5, **caractérisé en ce que** le tuyau d'appui (28a) présente une rigidité limitée, de sorte qu'il peut absorber et transmettre des forces de pression dans la direction longitudinale.

7. Système d'alimentation selon l'une quelconque des revendications 1 à 6, **caractérisé en ce que** le terminal de raccordement (8) présente une plaque avant (11) essentiellement verticale et l'extrémité libre de la conduite de dérivation (4) est guidée à travers un passage (12) dans la plaque avant (11).

8. Système d'alimentation selon l'une quelconque des revendications 5 à 7, **caractérisé en ce que** d'autres arrivées et/ou dérivations, en particulier pour un dialysat et/ou un concentré de dialyse, sont guidées dans le tuyau d'appui flexible (28a) parallèlement à la conduite de dérivation (4).

9. Système d'alimentation selon l'une quelconque des revendications 1 à 8, **caractérisé en ce que** le dispositif de guidage est agencé derrière un carénage (33) amovible.

10. Système d'alimentation pour appareils de dialyse selon l'une quelconque des revendications 1 à 9, **caractérisé en ce que** plusieurs terminaux de raccordement (8, 8') peuvent rouler le long du même rail de support (16),
chaque terminal de raccordement (8, 8') est associé à un dispositif de guidage qui guide au moins la conduite de dérivation (4, 4') associée destinée à l'eau pure.
